# EUROPEAN PATENT APPLICATION

(11) **EP 4 706 705 A1**
(43) Date of publication of application: **11.03.2026**
(21) Application number: 25199877.9
(22) Date of filing: 03.09.2025
(51) Int. Cl.: A61M 5/168, F16K 31/06, A61M 39/22, A61M 5/14

(54) **FAULT RECOVERY FOR STUCK VALVES**

(30) Priority: 05.09.2024 US 202463690905 P
(71) Applicant: Fresenius Kabi USA, LLC, Lake Zurich, IL 60047 (US)
(72) Inventor: POWERS, Benjamin G., Lake Zurich, 60047 (US); GONZALEZ, Lino A., Lake Zurich, 60047 (US)
(74) Representative: Maikowski & Ninnemann Patentanwälte Partnerschaft mbB

(57) **Abstract**

Systems and methods are provided for ensuring that an elastomeric seal of an armature of a solenoid valve of a fluid flow device is not stuck to an associated orifice prior to beginning a fluid flow procedure. A controller of the fluid flow device determines or is informed that a fluid flow assembly has been associated to the device while the armature is in a closed condition in which the elastomeric seal is seated against the orifice. Upon determining or being informed that the fluid flow assembly has been associated to the device, the controller executes a valve actuation sequence in which it controls a power source to deliver power to the solenoid valve so as to generate a magnetic field and attempt to cause the armature to move from the closed condition to an open condition in which the elastomeric seal is spaced from the orifice.

## Description

### Background

### Field of the Disclosure

The present subject matter relates to control of a solenoid valve having an elastomeric seal and an associated orifice. More particularly, the present subject matter relates to prevention of the elastomeric seal of a solenoid valve from sticking to an associated orifice.

### Description of Related Art

Fluid flow systems use variously configured valves to selectively allow and prevent fluid flow through a conduit associated with the valve, with fluid flow being allowed when the valve is in an open condition and with fluid flow being prevented when the valve is in a closed condition.

One common type of valve is a solenoid valve, which includes (among other components) a solenoid, an armature, a coil spring, and an orifice. The solenoid is typically comprised of a metallic filament arranged in a coil defining a central axis, with the solenoid being configured to generate a magnetic field when power is supplied to it from a power source. The armature is at least partially positioned within the solenoid (along the central axis) and at least partially formed of a ferromagnetic material that is configured to be moved along the central axis by a magnetic field generated by the solenoid. The coil spring is associated with the armature and urges the armature along the central axis toward the orifice. When no power is being supplied to the solenoid from the power source, the solenoid will not generate a magnetic field. When no magnetic field is being generated by the solenoid (or when the magnetic field generated by the solenoid is not of sufficient strength), the coil spring will cause the armature to contact the orifice (which is referred to herein as the armature or the solenoid valve being in a "closed condition"), thereby preventing fluid flow through a conduit associated with the valve. The armature includes an elastomeric seal element that is seated against the orifice when in the closed condition, rather than a rigid surface of the armature contacting the orifice.

When power is being supplied from the power source to the solenoid, the solenoid will generate a magnetic field that acts upon the armature. When sufficient power is being supplied to the solenoid, the magnetic field will be sufficiently strong to cause the armature to move away from the orifice, in opposition to the biasing force applied by the coil spring. With the armature (and, more particularly, the elastomeric seal of the armature) spaced away from the orifice (which is referred to herein as the armature or the solenoid valve being in an "open condition"), fluid is allowed to flow through the orifice and through the conduit associated with the valve. The selective application of power from the power source to the solenoid is controlled by a controller of the fluid flow system that is operatively coupled to the power source, with the controller commanding the power source to deliver power to the solenoid when fluid flow is to be allowed through the conduit associated with the valve and with the controller commanding the power source to not deliver power to the solenoid (or to not deliver a sufficient amount of power to cause the armature to move in opposition to the biasing force applied by the coil spring) when fluid flow through the conduit is to be prevented.

After long periods of inactivity (in which the solenoid valve is in its closed condition, with the elastomeric seal pressed against the orifice), the elastomeric seal can become stuck to the orifice and prevent the valve from opening when desired. Accordingly, it would be advantageous to be able to ensure that the elastomeric seal is not stuck to the orifice when a fluid flow procedure is to be carried out so as to ensure that the solenoid valve will work properly and as expected during the procedure.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a fluid flow device for use in combination with a fluid flow assembly includes a programmable controller, a power source operatively coupled to the controller, and a solenoid valve. The solenoid valve includes a solenoid electrically coupled to the power source and defining a central axis, with an armature at least partially positioned within the solenoid along the central axis, at least partially formed of a ferromagnetic material, and including an elastomeric seal. At least a portion of the elastomeric seal is seated against an orifice of the solenoid valve when the armature is in a closed condition, whereas the elastomeric seal is spaced away from the orifice when the armature is in an open condition. The armature is configured to be in the closed condition when the power source is not supplying power to the solenoid, with the controller being programmed to selectively control the power source to deliver power to the solenoid so as to generate a magnetic field that causes the armature to move along the central axis from the closed condition to the open condition. The controller is programmed to determine or to be informed that a fluid flow assembly has been associated to the fluid flow device while the armature is in the closed condition. Upon determining or being informed that the fluid flow assembly has been associated to the fluid flow device, the controller executes a valve actuation sequence in which it controls the power source to deliver power to the solenoid so as to generate a magnetic field and attempt to cause the armature to move from the closed condition to the open condition so as to unseat the elastomeric seal from the orifice.

In another aspect, a controller-implemented method is provided for ensuring that an elastomeric seal of an armature of a solenoid valve of a fluid flow device is not stuck to an associated orifice of the solenoid valve. The method includes the controller determining or being informed that a fluid flow assembly has been associated to the fluid flow device while the armature of the solenoid valve is in a closed condition in which at least a portion of the elastomeric seal is seated against the orifice. Upon determining or being informed that the fluid flow assembly has been associated to the fluid flow device, the controller executes a valve actuation sequence in which it controls a power source of the fluid flow device to deliver power to a solenoid of the solenoid valve so as to generate a magnetic field and attempt to cause the armature to move from the closed condition to an open condition in which the elastomeric seal is spaced from the orifice.

### Brief Description of the Drawings

Fig. 1 is a front elevational view of an exemplary fluid flow system according to an aspect of the present disclosure;
Figs. 2 and 3 are schematic views of a portion of the fluid flow device of Fig. 1, according to an exemplary embodiment; and
Figs. 4 and 5 are schematic views of a portion of the fluid flow device of Fig. 1, according to another exemplary embodiment.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

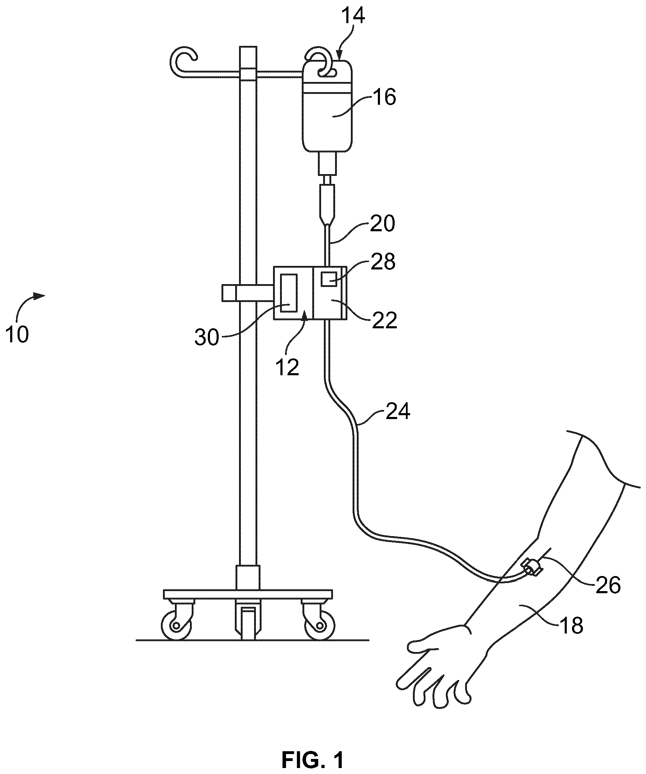

Fig. 1 shows an exemplary embodiment of a fluid flow system 10 embodying aspects of the present disclosure. The fluid flow system 10 includes a durable or reusable fluid flow device 12 and a fluid flow assembly 14 that is removably associated or mounted to the fluid flow device 12 in order to perform a fluid flow procedure. In the illustrated embodiment, the fluid flow system 10 is configured for delivery of a liquid from a source 16 to a recipient 18, which may include a liquid drug or medication being administered from a source container 16 to a living recipient 18, such as a human or animal. A fluid flow system of the type shown in Fig. 1 is described in greater detail in U.S. Patent Application Publication No. 2023/0126860, which is hereby incorporated herein by reference. It should, however, be understood that the principles described herein are not limited to use in administering medical fluids to living recipients, but are instead applicable to any fluid flow system in which a reusable or durable fluid flow device is used in combination with a removable fluid flow assembly (which may be either reusable or disposable).

In the embodiment of Fig. 1, the fluid flow assembly 14 is configured as a disposable or single-use administration set that includes the source container 16, an upstream tube 20 extending form the source container 16 to a cassette 22, and a downstream tube 24 extending from the cassette 22 to a recipient access device 26, such as a hollow needle or cannula that is placed into fluid communication with a vein of a patient. The cassette 22 provides for fluid communication between the upstream tube 20 and the downstream tube 24 and may be variously configured without departing from the scope of the present disclosure.

In Fig. 1, the cassette 22 of the fluid flow assembly 14 is associated or mounted to a component of the fluid flow device 12 that will be referred to herein as a "pump" 28, though it should be understood that the pump 28 may be configured to perform additional tasks other than pumping fluid from the source container 16 to the recipient 18. Additionally, while component 28 is referred to herein as a "pump," it should be understood that a fluid flow device according to the present disclosure does not require pump functionality, with fluid flow from a source to a recipient being achieved by other means, such as via gravity, for example.

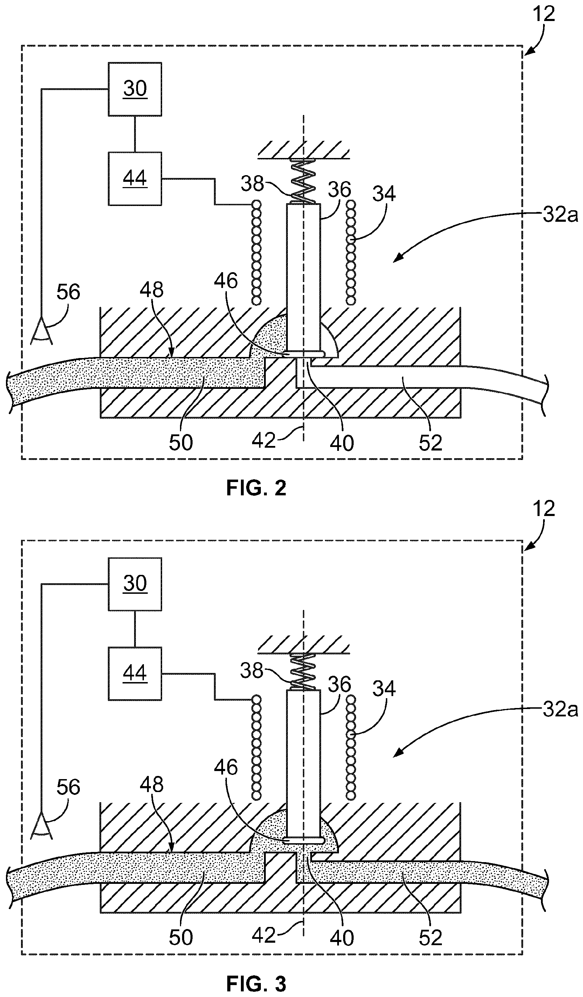

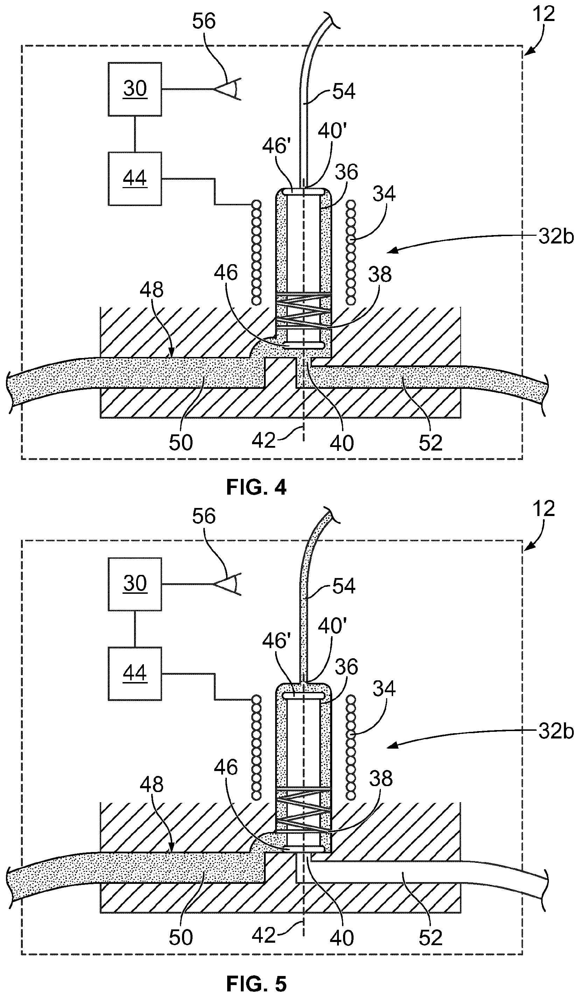

Regardless of whether the fluid flow device 12 includes pump functionality, it does include fluid flow control functionality, acting to selectively allow and prevent fluid flow during a procedure in which a fluid is conveyed from a source to a recipient. To that end, the fluid flow device 12 includes a programmable controller 30 that is programmed to control operation of a solenoid valve of the fluid flow device 12. A first exemplary embodiment of a solenoid valve 32a is shown in Figs. 2 and 3, while a second exemplary embodiment of a solenoid valve 32b is shown in Figs. 4 and 5. It should be understood that, while the term "fluid flow control functionality" encompasses use of a solenoid valve that directly acts to selectively allow and prevent the flow of a fluid being conveyed from a source to a recipient or destination, the term is not so limited. Instead, the term "fluid flow control functionality" also encompasses use of a solenoid valve that is actuated to selectively allow and prevent the flow of some other fluid that is present in a fluid flow path defined by the fluid flow device and/or the fluid flow assembly during a fluid flow procedure. For example, in one embodiment, a solenoid valve according to the present disclosure may be associated with a pneumatic circuit that conveys air through different regions of the fluid flow device, with the solenoid valve being actuated by the controller to selectively allow and prevent the flow of air through a conduit of the pneumatic circuit that is associated with the solenoid valve. In such an embodiment, the pneumatic circuit may be associated with a pump element of the fluid flow device, with the solenoid valve being actuated by the controller to selectively allow for and prevent the flow of air to the pump element in order to regulate the operation of the pump element during a fluid flow procedure.

In the embodiment of Figs. 2 and 3, the solenoid valve 32a is configured as a conventional two-way valve, with the solenoid valve 32a including a solenoid 34, an armature 36, a coil spring 38, and an orifice 40. The solenoid 34 is comprised of a metallic filament arranged in a coil defining a central axis 42, with the solenoid 34 being configured to generate a magnetic field when power is supplied to it from an associated power source 44 (which may be configured as a battery or the like). As shown in Figs. 2 and 3, in addition to the power source 44 being electrically coupled to the solenoid 34, the power source 44 is also operatively coupled to the controller 30.

The armature 36 (which may be generally cylindrical) is at least partially positioned within the solenoid 34 (along the central axis 42) and at least partially formed of a ferromagnetic material that is configured to be moved along the central axis 42 by a magnetic field generated by the solenoid 34. The coil spring 38 is associated with the armature 36 and urges the armature 36 along the central axis 42 (whether by directly or indirectly pushing or pulling the armature 36) toward the orifice 40. When no power is being supplied to the solenoid 34 from the power source 44, the solenoid 34 will not generate a magnetic field. When no magnetic field is being generated by the solenoid 34 (or when the magnetic field generated by the solenoid 34 is not of sufficient strength), the coil spring 38 will cause an elastomeric seal 46 of the armature 36 (which may be configured as a disc or a ring or the like) to contact the orifice 40, as shown in Fig. 2, which is referred to herein as the armature 36 or the solenoid valve 32a being in a "closed condition." The orifice 40 is associated with a conduit 48 extending through the solenoid valve 32a, with the orifice 40 connecting an upstream portion 50 of the conduit 48 to a downstream portion 52. As can be seen in Fig. 2, when the armature 36 is in the closed condition, with its elastomeric seal 46 seated against the orifice 40, flow through the orifice 40 will be prevented, thereby preventing fluid in one portion of the conduit 48 (illustrated in Fig. 2 as the upstream portion 50) from flowing through the orifice 40 and reaching the other portion of the conduit 48 (illustrated in Fig. 2 as the downstream portion 52).

When power is being supplied from the power source 44 to the solenoid 34, the solenoid 34 will generate a magnetic field that acts upon the armature 36. When sufficient power is being supplied to the solenoid 34, the magnetic field will be sufficiently strong to cause the armature 36 (including the elastomeric seal 46) to move away from the orifice 40, in opposition to the biasing force applied by the coil spring 38. With the armature 36 (and, more particularly, the elastomeric seal 46 of the armature 36) spaced away from the orifice 40 (which is shown in Fig. 3 and referred to herein as the armature 36 or the solenoid valve 32a being in an "open condition"), fluid is allowed to flow from one portion of the conduit 48 to the other portion via the orifice 40.

The selective application of power from the power source 44 to the solenoid 34 is controlled by the controller 30, with the controller 30 commanding the power source 44 to deliver power to the solenoid 34 when fluid flow is to be allowed through the conduit 48 and with the controller 30 commanding the power source 44 to not deliver power to the solenoid 34 (or to not deliver a sufficient amount of power to cause the armature 36 to move in opposition to the biasing force applied by the coil spring 38) when fluid flow through the conduit 48 is to be prevented. The controller 30 may be variously configured without departing from the scope of the present disclosure. In one exemplary embodiment, the controller 30 comprises a main processing unit (MPU), which can comprise, e.g., a Pentium^{™} type microprocessor made by Intel Corporation, although other types of conventional microprocessors can be used.

Figs. 4 and 5 illustrate a solenoid valve 32b that is similar to the solenoid valve 32a of Figs. 2 and 3 (with the same reference numbers being applied to corresponding components), but is instead configured as a conventional three-way valve. As in the embodiment of Figs. 2 and 3, the solenoid valve 32b of Figs. 4 and 5 includes a solenoid 34, an armature 36, a coil spring 38, and an orifice 40. However, rather than defining a single orifice 40, the solenoid valve 32b of Figs. 4 and 5 also defines a second orifice 40'. As can be seen in Figs. 4 and 5, one of the orifices 40 is positioned adjacent to one end of the armature 36, while the other orifice 40' is positioned adjacent to the other end of the armature 36. On account of the solenoid valve 32b employing two orifices 40 and 40', the armature 36 includes a pair of elastomeric seals 46 and 46', with one elastomeric seal 46' positioned to be selectively moved into contact with one of orifices 40' (Fig. 4) and the other elastomeric seal 46 positioned to be selectively moved into contact with the other orifice 40 (Fig. 5).

In the illustrated embodiment, the coil spring 38 is configured to bias the armature 36 into the raised position shown in Fig. 4, in which the upper elastomeric seal 46' is seated against the upper orifice 40', thereby preventing fluid communication with an upper conduit portion 54. Even though the lower orifice 40 is unblocked and fluid communication is allowed between the upstream and downstream conduit portions 50 and 52, this position of the armature 36 will be referred to herein as the "closed condition," on account of it being the position to which the coil spring 38 biases the armature 36 and the position in which the armature 36 will be when the solenoid 38 is not generating a magnetic field. In one embodiment, the upper conduit portion 54 may be connected to a functional component of the fluid flow device 12 (e.g., a pump element) that is configured to be actuated during a fluid flow procedure by fluid being conveyed into the upper conduit portion 54, such that the armature position of Fig. 4 may be understood as a "closed condition" to the extent that fluid communication with the functional component is closed or prevented. In such an embodiment, the upstream and downstream conduit portions 50 and 52 may be connected to a fluid reservoir and allowed to initially be in fluid communication with each other without consequence or, if fluid flow between the upstream and downstream conduit portions 50 and 52 is to be initially prevented, another valve associated with one of them may be closed to prevent fluid from being present in either portion 50, 52 when the armature 36 is in the closed condition of Fig. 4.

As in the embodiment of Figs. 2 and 3, when power is being supplied from the power source 44 to the solenoid 34 of Fig. 4, the solenoid 34 will generate a magnetic field that acts upon the armature 36. When sufficient power is being supplied to the solenoid 34, the magnetic field will be sufficiently strong to cause the armature 36 (including the upper elastomeric seal 46') to move downwardly away from the upper orifice 40', in opposition to the biasing force applied by the coil spring 38. With the armature 36 (and, more particularly, the upper elastomeric seal 46' of the armature 36) spaced away from the upper orifice 40' (Fig. 5), fluid is allowed to flow from the upstream conduit portion 50 to the upper conduit portion 54, while the downward movement of the armature 36 will bring the lower elastomeric seal 46 into contact with the lower orifice 40. Consistent with the above description of the closed condition of Fig. 4, even though the lower orifice 40 is blocked by the lower elastomeric seal 46 and fluid communication is prevented between the upstream and downstream conduit portions 50 and 52, this position of the armature 36 will be referred to herein as the "open condition," on account of it being the position to which the armature 36 will be when the solenoid 34 is generating a relatively strong magnetic field.

Regardless of the particular configuration of the solenoid valve, and as explained above, after long periods of inactivity (in which the solenoid valve is in its closed condition, with the elastomeric seal pressed against the orifice), the elastomeric seal can become stuck to the orifice and prevent the valve from opening when desired. According to an aspect of the present disclosure, the controller 30 is programmed to execute a valve actuation sequence prior to beginning a fluid flow procedure, with the valve actuation sequence ensuring that the elastomeric seal of a solenoid valve is not stuck to an associated orifice when the procedure is initiated. In particular, the controller 30 is programmed to begin the valve actuation sequence when a removable fluid flow assembly 14 has been associated to the fluid flow device 12. The controller 30 may be informed (e.g., by an operator) that the fluid flow assembly 14 has been associated to the fluid flow device 12 or may itself determine that the fluid flow assembly 14 has been associated to the fluid flow device 12.

If the controller 30 is programmed to determine when the fluid flow assembly 14 has been associated to the fluid flow device 12, it may do so in any suitable fashion. For example, the fluid flow device 12 may include one or more sensors 56 operatively coupled to the controller 30, with each sensor 56 being configured to transmit signals to the controller 30 that are indicative of whether the fluid flow assembly 14 has been associated to the fluid flow device 12. In one embodiment, the sensor 56 may be configured as a weight scale associated with a hook or the like from which a source container 16 of the fluid flow assembly 14 is suspended, with the controller 30 determining that the fluid flow assembly 14 has been associated to the fluid flow device 12 upon receiving signals from the weight scale indicative of the presence of the source container 16. In another embodiment, the sensor 56 may be configured as an optical sensor configured to determine when the fluid flow assembly 14 has been associated to the fluid flow device 12. In the embodiment of Fig. 1, such an optical sensor may include a light source and a light detector that are spaced apart by a cavity configured to receive a portion of the cassette 22 when the fluid flow assembly 14 has been associated to the pump 28 of the fluid flow device 12. When a cassette 22 is not present in the cavity, a certain amount of the light transmitted by the light source will be received by the light detector, with the light detector transmitting a high-voltage signal to the controller 30 which indicates that the fluid flow assembly 14 has not yet been associated to the fluid flow device 12. When a portion of the cassette 22 is present in the cavity (and positioned between the light source and the light detector), that portion of the cassette 22 will prevent some or all of the light transmitted by the light source from reaching the light detector. In that case, the light detector will transmit a lower-voltage (or zero-voltage) signal to the controller 30, thus indicating to the controller 30 that the fluid flow assembly 14 has been associated to the fluid flow device 12.

Regardless of whether the controller 30 is informed or determines that the fluid flow assembly 14 has been associated to the fluid flow device 12, when the controller 30 becomes aware that the fluid flow assembly 14 has been associated to the fluid flow device 12, it will immediately proceed to execute the valve actuation sequence. The exact nature of the valve actuation sequence (e.g., its duration, the number of steps executed by the controller 30, the particular components of the fluid flow device 12 that are controlled by the controller 30, etc.) may vary without departing from the scope of the present disclosure, provided that it is calculated to ensure that the elastomeric seal of an armature of a solenoid valve is not stuck or adhered to an associated orifice of the solenoid valve. Once the controller 30 has successfully completed the valve actuation sequence, it may allow for the fluid flow procedure to begin. On the other hand, if the controller 30 determines (e.g., based on a signal transmitted from the solenoid valve to the controller 30) that the valve actuation sequence has not been successful and that an elastomeric seal of the solenoid valve remains stuck to an associated orifice (or that there is some other error in the functioning of the solenoid valve during the valve actuation sequence), the controller 30 may generate an alert or otherwise advise an operator and prevent the execution of a fluid flow procedure until the irregularity has been resolved.

In one embodiment, the valve actuation sequence includes a plurality of stages, with each stage taking a different approach to dislodging an elastomeric seal that has become stuck to an associated orifice. In an exemplary first stage of such a multi-stage valve actuation sequence, the controller 30 controls the power source to deliver a long-duration, high-voltage pulse of power to the solenoid so as to cause the solenoid to generate a magnetic field and attempt to move the armature from its closed condition to its open condition and then retain the armature in the open condition during the entirety of the first stage. In an exemplary second stage of a multi-stage valve actuation sequence, the controller 30 controls the power source to alternate between: (1) delivering high-voltage power to the solenoid so as to cause the solenoid to generate a magnetic field and attempt to move the armature from the closed condition to the open condition and (2) delivering no power to the solenoid so as to allow the armature to move from the open condition to the closed condition. The two stages may be executed once each or they may be repeated. In an alternative embodiment of a two-stage valve actuation sequence, the second stage may be executed before the first stage. In alternative embodiments, a single-stage valve actuation sequence may implement only the first stage or only the second stage or a multi-stage valve actuation sequence may implement the first and second stages along with one or more additional stage (in any order).

Turning now more particularly to an exemplary first stage of a valve actuation sequence, the controller 30 may attempt to dislodge an elastomeric seal from an associated orifice by controlling the power source to deliver a long-duration, high-voltage pulse of power to the solenoid of a solenoid valve so as to cause the solenoid to generate a magnetic field and attempt to move the armature from its closed condition to its open condition and then retain the armature in the open condition for the remainder of the first stage. The duration and magnitude of the "high-voltage" pulse of power delivered from the power source to the solenoid during the first stage may vary without departing from the scope of the present disclosure. For example, in one embodiment, the first stage may last for one second, with the controller 30 controlling the power source to deliver 12 volts of power (sufficient to cause the solenoid to generate a magnetic field that is strong enough to cause the armature to move from its closed condition to its open condition) for the entire one second.

In other embodiments, the controller 30 may need to control operation of the power source during the first stage in a way that effectively circumvents the logic of an associated drive circuit when operating to cause the solenoid valve to move to and remain in its open condition. For example, in one known system, the logic of a drive circuit is such that a solenoid valve will be driven with power applied in a "hit and hold" approach when the valve input signal is kept in an "on" position. By such an approach, a "high-voltage" pulse of power is briefly supplied to the solenoid ("hit"), followed by a longer supply of power at a lower voltage ("hold"). The "high-voltage" pulse of power is intended to result in a relatively strong magnetic field that initially moves the armature from the closed condition to the open condition and is referred to as the "hit" portion of the "hit and hold" approach because of its high voltage and short duration. Once the armature has been moved to its open condition, a weaker magnetic field will be sufficient to retain it in the open condition, such that power at a lower voltage may be supplied to the solenoid for as long as the magnetic field is to hold the armature in place (which is why this may be referred to as the "hold" portion of the "hit and hold" approach).

In one embodiment, the "high-voltage" pulse of power applied during the "hit" phase is 12 volts, while the lower voltage power applied during the "hold" phase is between 6.8 and 9 volts, with the "hit" phase lasting for 12 msec for a two-way valve and 50 msec for a three-way valve. On account of the logic of the drive circuit, once the "hit" timer (of 12 msec for a two-way valve and 50 msec for a three-way valve) has elapsed, the "hold" phase will begin and last for as long as the solenoid valve is to remain in its open condition. It will, thus, be seen that this logic of the drive circuit would prevent continuous operation of the power source to deliver "high-voltage" power to the solenoid for the duration of the first stage of a valve actuation sequence (which may last for one second, in one embodiment). In order to circumvent such logic of the drive circuit and allow for "high-voltage" power to effectively be supplied to the solenoid for a longer period of time, the controller 30 may act during the first stage of the valve actuation sequence to control the power source to alternately deliver "high-voltage" power to the solenoid for a first duration and deliver no power to the solenoid for a second duration. The first duration is selected to be shorter than the "hit" timer, thereby preventing the logic of the drive circuit from advancing to a "hold" phase in which a lower level of power is supplied to the solenoid. This may include the controller 30 controlling the power source to deliver "high-voltage" power to the solenoid of a two-way valve for 10 msec (compared to the 12 msec duration of the "hit" timer) and to deliver "high-voltage" power to the solenoid of a three-way valve for 40 msec (compared to the 50 msec duration of the "hit" timer). The "hit" timer for a three-way valve may be longer than the "hit" timer for a two-way valve due to three-way valves typically taking longer to physically actuate (i.e., move the armature from the closed condition to the open condition) for various reasons (e.g., differences in the solenoid, the spring force of the coil spring, and the orifice sizes of the two valve types).

Ending the application of "high-voltage" power before the "hit" timer would elapse causes the "hit" timer to be reset for when "high-voltage" power is to be later applied to the solenoid. Between successive applications of "high-voltage" power, the power source is turned off for a second duration (as explained above) to allow for the "hit" timer to be reset. Typically, when no power is being supplied from the power source to the solenoid, the solenoid will not generate a magnetic field, thus allowing the armature to move from the open condition to the closed condition. However, when the power is turned off very briefly (e.g., for a "second duration" of one msec for either a two-way valve or a three-way valve) and then turned back on again, there will not be sufficient time for the current in the solenoid to decay (due to the coil inductance), such that the solenoid will continue to generate a strong magnetic field (as if the "high-voltage" power were still being supplied to the solenoid) during the short time that the power source is turned off, and the armature will remain in its open condition. Thus, the armature will still be in its open condition when the controller 30 commands the power source to again supply "high-voltage" power to the solenoid, thereby circumventing the logic of the drive circuit and allowing the controller 30 to effectively cause "high-voltage" power to be applied to the solenoid during the entirety of the first stage of the valve actuation sequence.

While such a first stage of a valve actuation sequence will frequently be effective in dislodging an elastomeric seal from an associated orifice, it has been found that, in some situations, an elastomeric seal will be so strongly stuck to an associated orifice that even a long-duration, high-voltage pulse of power to the solenoid (as is implemented during the first stage of this exemplary valve actuation sequence) will not be sufficient to dislodge the elastomeric seal, which is why a second stage may be implemented following completion of the first stage. As described above, during an exemplary second stage of a valve actuation sequence, the controller 30 controls the power source to alternate between: (1) delivering high-voltage power to the solenoid so as to cause the solenoid to generate a magnetic field and attempt to move the armature from the closed condition to the open condition and (2) delivering no power to the solenoid so as to allow the armature to move from the open condition to the closed condition. These alternating phases of attempting to move the armature to its open condition and then allowing the solenoid valve to return to its closed condition may be repeated (e.g., being repeated ten times), such that a second stage of this type may be understood as "jiggling" the armature to attempt to unstick an elastomeric seal from an associate orifice. In one embodiment, the "high-voltage" power applied by the power source during the first phase of this second stage is the same as the power supplied to the solenoid during the first stage of the valve actuation sequence (which is 12 volts in this exemplary embodiment), which is high enough to cause the solenoid to generate a magnetic field that should cause the armature to move from its closed condition to its open condition. The duration of each phase of the second stage of the valve actuation sequence may vary without departing from the scope of the present disclosure, provided that the first phase is long enough to allow for the armature move from its closed condition to its open condition and the second phase is long enough to allow for the armature to move back to its closed condition. For example, in one embodiment, it has been found that deactivating the power source for 40 msec (whether for a two-way valve or a three-way valve) is sufficiently long to allow for the current in the solenoid to decay and, thus, allow for the armature to move from the open condition to its closed condition, without being unnecessarily long.

Again, depending on the configuration of the fluid flow device, the controller 30 may need to control operation of the power source during the second stage in a way that is informed by the logic of an associated drive circuit. For example, in the known system described above in the discussion of the first stage of the valve actuation sequence, a "hit" timer limits the maximum time that "high-voltage" power may be supplied from the power source to the solenoid to 12 msec for a two-way valve and 50 msec for a three-way valve. When practicing the principles described herein with such a system, it may be advantageous for the first phase of the second stage of the valve actuation sequence (during which "high-voltage" power is supplied to the solenoid) to last for 10 msec for a two-way valve and 40 msec for a three-way valve (which is the same as the above-described "first durations" of application of "high-voltage" power during the first stage of the valve actuation sequence), so as to allow for the "hit" timer to reset, rather than allowing for the logic of the drive circuit to automatically advance to the application of power at a lower voltage when the "hit" timer has elapsed.

Once both stages of the valve actuation sequence have been completed, they may be repeated one or more times to better ensure that all of the solenoid valves of the fluid flow device are working properly when a fluid flow procedure using the fluid flow assembly is begun. This is also true for any other valve actuation sequence according to the present disclosure - once all of the unique stages have been completed once, one or more of them may be repeated one or more times in order to better ensure proper valve operation.

### Aspects

Aspect 1. A fluid flow device for use in combination with a fluid flow assembly, the fluid flow device comprising: a programmable controller; a power source operatively coupled to the controller; and a solenoid valve including a solenoid electrically coupled to the power source and defining a central axis, an armature at least partially positioned within the solenoid along the central axis, at least partially formed of a ferromagnetic material, and including an elastomeric seal, and an orifice, wherein at least a portion of the elastomeric seal is seated against the orifice when the armature is in a closed condition, the elastomeric seal is spaced away from the orifice when the armature is in an open condition, the armature is configured to be in the closed condition when the power source is not supplying power to the solenoid, the controller is programmed to selectively control the power source to deliver power to the solenoid so as to generate a magnetic field that causes the armature to move along the central axis from the closed condition to the open condition, and the controller is programmed to determine or to be informed that a fluid flow assembly has been associated to the fluid flow device while the armature is in the closed condition and, upon determining or being informed that the fluid flow assembly has been associated to the fluid flow device, execute a valve actuation sequence in which the controller controls the power source to deliver power to the solenoid so as to generate said magnetic field and attempt to cause the armature to move from the closed condition to the open condition so as to unseat said at least a portion of the elastomeric seal from the orifice.

Aspect 2. The fluid flow device of Aspect 1, wherein said valve actuation sequence includes a plurality of stages.

Aspect 3. The fluid flow device of Aspect 2, wherein said valve actuation sequence includes a first stage in which the controller controls the power source to deliver a long-duration, high-voltage pulse of power to the solenoid so as to cause the solenoid to generate said magnetic field and attempt to move the armature from the closed condition to the open condition and retain the armature in the open condition during the entirety of the first stage.

Aspect 4. The fluid flow device of Aspect 3, wherein said long-duration, high-voltage pulse of power has a duration of approximately one second.

Aspect 5. The fluid flow device of Aspect 3, wherein said long-duration, high-voltage pulse of power includes the power source alternately delivering high-voltage power to the solenoid for a first duration and delivering no power to the solenoid for a second duration, and the second duration is sufficiently short that the armature is not allowed to move from the open condition to the closed condition.

Aspect 6. The fluid flow device of Aspect 2, wherein said valve actuation sequence includes a second stage in which the controller controls the power source to alternately deliver high-voltage power to the solenoid so as to cause the solenoid to generate said magnetic field and attempt to move the armature from the closed condition to the open condition, and deliver no power to the solenoid so as to allow the armature to move from the open condition to the closed condition.

Aspect 7. The fluid flow device of Aspect 6, wherein high-voltage power is delivered to the solenoid during the second stage for a duration that is equal to a duration during which power is not delivered to the solenoid during the second stage.

Aspect 8. The fluid flow device of Aspect 1, wherein said valve actuation sequence includes a first stage in which the controller controls the power source to deliver a long-duration, high-voltage pulse of power to the solenoid so as to cause the solenoid to generate said magnetic field and attempt to move the armature from the closed condition to the open condition and retain the armature in the open condition during the first stage, and a second stage in which the controller controls the power source to alternately deliver high-voltage power to the solenoid so as to cause the solenoid to generate said magnetic field and attempt to move the armature from the closed condition to the open condition, and deliver no power to the solenoid so as to allow the armature to move from the open condition to the closed condition.

Aspect 9. The fluid flow device of any one of the preceding Aspects, wherein the solenoid valve is configured as a two-way valve.

Aspect 10. The fluid flow device of any one of Aspects 1-8, wherein the solenoid valve is configured as a three-way valve.

Aspect 11. A controller-implemented method for ensuring that an elastomeric seal of an armature of a solenoid valve of a fluid flow device is not stuck to an associated orifice of the solenoid valve, the method comprising: determining or being informed that a fluid flow assembly has been associated to the fluid flow device while the armature of the solenoid valve is in a closed condition in which at least a portion of the elastomeric seal is seated against the orifice, and upon determining or being informed that the fluid flow assembly has been associated to the fluid flow device, executing a valve actuation sequence in which the controller controls a power source of the fluid flow device to deliver power to a solenoid of the solenoid valve so as to generate a magnetic field and attempt to cause the armature to move from the closed condition to an open condition in which the elastomeric seal is spaced from the orifice.

Aspect 12. The method of Aspect 11, wherein said valve actuation sequence includes a plurality of stages.

Aspect 13. The method of Aspect 12, wherein said valve actuation sequence includes a first stage in which the controller controls the power source to deliver a long-duration, high-voltage pulse of power to the solenoid so as to cause the solenoid to generate said magnetic field and attempt to move the armature from the closed condition to the open condition and retain the armature in the open condition during the entirety of the first stage.

Aspect 14. The method of Aspect 13, wherein said long-duration, high-voltage pulse of power has a duration of approximately one second.

Aspect 15. The method of Aspect 13, wherein said long-duration, high-voltage pulse of power includes the power source alternately delivering high-voltage power to the solenoid for a first duration and delivering no power to the solenoid for a second duration, and the second duration is sufficiently short that the armature is not allowed to move from the open condition to the closed condition.

Aspect 16. The method of Aspect 12, wherein said valve actuation sequence includes a second stage in which the controller controls the power source to alternately deliver high-voltage power to the solenoid so as to cause the solenoid to generate said magnetic field and attempt to move the armature from the closed condition to the open condition, and deliver no power to the solenoid so as to allow the armature to move from the open condition to the closed condition.

Aspect 17. The method of Aspect 16, wherein high-voltage power is delivered to the solenoid during the second stage for a duration that is equal to a duration during which power is not delivered to the solenoid during the second stage.

Aspect 18. The method of Aspect 11, wherein said valve actuation sequence includes a first stage in which the controller controls the power source to deliver a long-duration, high-voltage pulse of power to the solenoid so as to cause the solenoid to generate said magnetic field and attempt to move the armature from the closed condition to the open condition and retain the armature in the open condition during the first stage, and a second stage in which the controller controls the power source to alternately deliver high-voltage power to the solenoid so as to cause the solenoid to generate said magnetic field and attempt to move the armature from the closed condition to the open condition, and deliver no power to the solenoid so as to allow the armature to move from the open condition to the closed condition.

Aspect 19. The method of any one of Aspects 11-18, wherein the solenoid valve is configured as a two-way valve.

Aspect 20. The method of any one of Aspects 11-18, wherein the solenoid valve is configured as a three-way valve.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A fluid flow device (12) for use in combination with a fluid flow assembly (14), the fluid flow device (12) comprising:
a programmable controller (30);
a power source (44) operatively coupled to the controller (30); and
a solenoid valve (32a, 32b) including
a solenoid (34) electrically coupled to the power source (44) and defining a central axis (42),
an armature (36) at least partially positioned within the solenoid (34) along the central axis (42), at least partially formed of a ferromagnetic material, and including an elastomeric seal (46, 46'), and
an orifice (40, 40'), wherein
at least a portion of the elastomeric seal (46, 46') is seated against the orifice (40, 40') when the armature (36) is in a closed condition,
the elastomeric seal (46, 46') is spaced away from the orifice (40, 40') when the armature (36) is in an open condition,
the armature (36) is configured to be in the closed condition when the power source (44) is not supplying power to the solenoid (34),
the controller (30) is programmed to selectively control the power source (44) to deliver power to the solenoid (34) so as to generate a magnetic field that causes the armature (36) to move along the central axis (42) from the closed condition to the open condition, and
the controller (30) is programmed to determine or to be informed that a fluid flow assembly (14) has been associated to the fluid flow device (12) while the armature (36) is in the closed condition and, upon determining or being informed that the fluid flow assembly (14) has been associated to the fluid flow device (12), execute a valve actuation sequence in which the controller (30) controls the power source (44) to deliver power to the solenoid (34) so as to generate said magnetic field and attempt to cause the armature (36) to move from the closed condition to the open condition so as to unseat said at least a portion of the elastomeric seal (46, 46') from the orifice (40, 40').

2. A controller-implemented method for ensuring that an elastomeric seal (46, 46') of an armature (36) of a solenoid valve (32a, 32b) of a fluid flow device (12) is not stuck to an associated orifice (40, 40') of the solenoid valve (32a, 32b), the method comprising:
determining or being informed that a fluid flow assembly (14) has been associated to the fluid flow device (12) while the armature (36) of the solenoid valve (32a, 32b) is in a closed condition in which at least a portion of the elastomeric seal (46, 46') is seated against the orifice (40, 40'), and
upon determining or being informed that the fluid flow assembly (14) has been associated to the fluid flow device (12), executing a valve actuation sequence in which the controller (30) controls a power source (44) of the fluid flow device (12) to deliver power to a solenoid (34) of the solenoid valve (32a, 32b) so as to generate a magnetic field and attempt to cause the armature (36) to move from the closed condition to an open condition in which the elastomeric seal (46, 46') is spaced from the orifice (40, 40').

3. The fluid flow device (12) of claim 1 or the method of claim 2, wherein said valve actuation sequence includes a plurality of stages.

4. The fluid flow device (12) or method of claim 3, wherein said valve actuation sequence includes a first stage in which the controller (30) controls the power source (44) to deliver a long-duration, high-voltage pulse of power to the solenoid (34) so as to cause the solenoid (34) to generate said magnetic field and attempt to move the armature (36) from the closed condition to the open condition and retain the armature (36) in the open condition during the entirety of the first stage.

5. The fluid flow device (12) or method of claim 4, wherein said long-duration, high-voltage pulse of power has a duration of approximately one second.

6. The fluid flow device (12) or method of claim 4, wherein
said long-duration, high-voltage pulse of power includes the power source (44) alternately delivering high-voltage power to the solenoid (34) for a first duration and delivering no power to the solenoid (34) for a second duration, and
the second duration is sufficiently short that the armature (36) is not allowed to move from the open condition to the closed condition.

7. The fluid flow device (12) or method of claim 3, wherein said valve actuation sequence includes a second stage in which the controller (30) controls the power source (44) to alternately
deliver high-voltage power to the solenoid (34) so as to cause the solenoid (34) to generate said magnetic field and attempt to move the armature (36) from the closed condition to the open condition, and
deliver no power to the solenoid (34) so as to allow the armature (36) to move from the open condition to the closed condition.

8. The fluid flow device (12) or method of claim 7, wherein high-voltage power is delivered to the solenoid (34) during the second stage for a duration that is equal to a duration during which power is not delivered to the solenoid (34) during the second stage.

9. The fluid flow device (12) of claim 1 or the method of claim 2, wherein said valve actuation sequence includes
a first stage in which the controller (30) controls the power source (44) to deliver a long-duration, high-voltage pulse of power to the solenoid (34) so as to cause the solenoid (34) to generate said magnetic field and attempt to move the armature (36) from the closed condition to the open condition and retain the armature (36) in the open condition during the first stage, and
a second stage in which the controller (30) controls the power source (44) to alternately
deliver high-voltage power to the solenoid (34) so as to cause the solenoid (34) to generate said magnetic field and attempt to move the armature (36) from the closed condition to the open condition, and
deliver no power to the solenoid (34) so as to allow the armature (36) to move from the open condition to the closed condition.

10. The fluid flow device (12) of any one of claims 1 and 3-9 or the method of any one of claims 2-9, wherein the solenoid valve (32a) is configured as a two-way valve.

11. The fluid flow device (12) of any one of claims 1 and 3-9 or the method of any one of claims 2-9, wherein the solenoid valve (32b) is configured as a three-way valve.
